# EUROPEAN PATENT SPECIFICATION

(11) **EP 3 636 817 B1**
(45) Date of publication and mention of the grant of the patent: **07.06.2023**
(21) Application number: 19202048.5
(22) Date of filing: 08.10.2019
(51) Int. Cl.: D04H 1/728, G01N 21/78, G01N 31/22

(54) **METHOD OF FABRICATING A COLORIMETRIC GAS SENSOR BASED ON NANOFIBER YARN FOR GAS INDICATION INCLUDING IONIC LIQUIDS AND CHROMOGENIC DYES**
VERFAHREN ZUR HERSTELLUNG EINES KOLORIMETRISCHEN GASSENSORS AUF DER BASIS VON NANOFASERGARN ZUR GASANZEIGE MIT IONISCHEN FLÜSSIGKEITEN UND CHROMOGENEN FARBSTOFFEN
MÉTHODE DE FABRICATION D'UN CAPTEUR DE GAZ COLORIMÉTRIQUE BASÉ SUR UN FIL DE NANOFIBRES POUR L'INDICATION DE GAZ COMPRENANT DES LIQUIDES IONIQUES ET DES LES COLORANTS CHROMOGÈNES

(30) Priority: 08.10.2018 KR 20180119707
(43) Date of publication of application: 15.04.2020
(73) Proprietor: Korea Advanced Institute Of Science And Technology, Daejeon, 34141 (KR)
(72) Inventor: Kim, Il-Doo, 34141 Daejeon (KR); Kim, Dong-Ha, 34141 Daejeon (KR); Kim, Chanhoon, 34141 Daejeon (KR)
(74) Representative: Keller Schneider Patent- und Markenanwälte AG

(56) References cited:
- WO-A1-2018/139899
- SIBEL AYDOGDU ET AL: "Optical COSensing with Ionic Liquid Doped Electrospun Nanofibers", JOURNAL OF FLUORESCENCE, KLUWER ACADEMIC PUBLISHERS-PLENUM PUBLISHERS, NE, vol. 21, no. 2, 14 October 2010 (2010-10-14), pages 607-613, XP019909931, ISSN: 1573-4994, DOI: 10.1007/S10895-010-0748-4
- KAMALAKANTA BEHERA ET AL: "Ionic Liquid-Based Optical and Electrochemical Carbon Dioxide Sensors", SENSORS, vol. 15, no. 12, 4 December 2015 (2015-12-04), pages 30487-30503, XP055403608, DOI: 10.3390/s151229813
- AMUTHA CHINNAPPAN ET AL: "An overview of electrospun nanofibers and their application in energy storage, sensors and wearable/flexible electronics", JOURNAL OF MATERIALS CHEMISTRY C, vol. 5, no. 48, 1 January 2017 (2017-01-01), pages 12657-12673, XP055671084, GB ISSN: 2050-7526, DOI: 10.1039/C7TC03058D
- ONGUN MERVE ZEYREK ET AL: "Enhanced stability of ruthenium complex in ionic liquid doped electrospun fibers", SENSORS AND ACTUATORS B: CHEMICAL, ELSEVIER BV, NL, vol. 183, 23 March 2013 (2013-03-23), pages 11-19, XP028549873, ISSN: 0925-4005, DOI: 10.1016/J.SNB.2013.03.060

## Description

### BACKGROUND OF THE INVENTION

### 1. Technical Field

The present invention generally relates to a method of fabricating a colorimetric gas sensor using a complex polymer nanofiber structure for yarn-based gas indication in which color change dyes having a varying color are functionalized into a nanofiber through a reaction between ionic liquids and specific gas molecules.

### 2. Description of the Related Art

A gas sensor is a detection device capable of measuring the concentration of specific gas molecules in the atmosphere. Loss of lives can be prevented by early detection the leakage of noxious gases in factories using the gas sensor. Furthermore, exposure to a contaminated environment can be minimized by measuring quality of indoor and outdoor air in the real life using the gas sensor.

WO 2018/139899 A1 discloses a method for fabricating a nanofiber based gas sensor comprising an ionic liquid.

Furthermore, recently, as an interest in healthcare suddenly increases, research of an exhaled breath sensor for early diagnosing whether a specific disease is present by precisely detecting a small amount of biomarker gas included in the human's exhaled breath is also carried out actively. If a person has a specific disease, a specific biomarker substance is generated or the existing biomarker substance is generated more within the human body. In this case, the generated biomarker substances are discharged as an exhaled breath through the lung in a gas form. The exhaled breath of the human body includes various biomarker gases, such as hydrogen sulfide, acetone, toluene, and ammonia. Such gases are reported as biomarkers for halitosis, diabetes, lung cancer, and kidney disorder.

Widely used gas sensors include gas chromatography, a variable resistive-type gas sensor, and a colorimetric gas sensor. The sensors are selectively used depending on their usages or environments. The gas chromatography enables precise qualitative and quantitative analysis compared to other gas sensors, but has disadvantages in that the price is high, the size is large, and a long analysis time is taken. The variable resistive-type gas sensor uses a semiconductor substance based on metal oxides, and performs quantitative and qualitative analysis on a target gas by measuring a variation in electric resistance value upon exposure to the target gas, which varies on a surface of a metal oxide semiconductor in an adsorption and desorption process. The resistive-type gas sensor has advantage in that it has a simple construction and can be easily carried, but has disadvantages in that the type of gas capable of being measured is limited and sensitivity characteristics and a concentration of the limit of detection are high compared to the gas chromatography. Furthermore, such a gas sensor has a disadvantage in that additional it requires power consumption because a heater for an operation at a high temperature and a circuit for detecting electric resistance in real-time are necessary.

### SUMMARY OF THE INVENTION

The present invention provides a method for fabricating a colorimetric gas sensor based on a nanofiber yarn for gas indication, wherein color change dyes causing a color change through adsorption and a reaction with specific gas molecules that are not desorbed from the product during use, an area where the color change dye is exposed to the air is maximized, ionic liquids and the color change dye are included in a one-dimensional (1-D) nanofiber structure at the same time, and such nanofiber structures are tangled up together in a bundle form to form a three-dimensional (3-D) network structure. The method of the present invention is defined in claim 1. The gas sensor described in here is not part of the present invention.

The colorimetric gas sensor obtained by the method of the present invention can improve the adsorption property of gas because ionic liquids and color change dyes are anchored within nanofibers and on a surface of the nanofiber to increase the solubility of specific gas molecules through the ionic liquids, can reduce an environmental pollution problem by minimizing color change dye content causing a color change through a surface chemical reaction with gas molecules adsorbed on a surface, and can detect an ultra-small amount of specific gas molecules.

In an aspect, an electro-spinning solution in which ionic liquids and color change dyes are mixed with a polymer solution having a polymer dissolved in a solvent is fabricated. After the electro-spinning solution is stirred at a temperature of a melting point or higher of the color change dyes, a distributed electro-spinning solution in which the color change dyes have been re-crystallized into fine crystals and distributed through a quenching process is fabricated in accord with the method of the present invention. Through the electro-spinning process using the electro-spinning solution, a complex polymer nanofiber structure in which the ionic liquids and the color change dyes have been uniformly anchored can be fabricated. In this case, an independent type sensor of a thread form can be fabricated by winding the complex polymer nanofiber in a yarn structure through a multi-electro-spinning scheme and the rotation of a current collector.

In another aspect, a method of fabricating a gas sensor includes (a) fabricating a mixed solution in which ionic liquids and color change dyes are mixed with a polymer solution in which a polymer is dissolved in a solvent; (b) dissolving the ionic liquids and the color change dyes within the mixed solution through a high-temperature stirring process; (c) fabricating an electro-spinning solution containing dyes coagulated into fine crystals through a quenching process for the mixed solution in which the ionic liquids and the color change dyes have been dissolved; (d) fabricating a one-dimensional (1-D) polymer nanofiber in which the ionic liquids and the color change dyes have been anchored using a dual electro-spinning process and fabricating the 1-D polymer nanofiber into a nanofiber having a 3-D network structure of a yarn shape; and (e) winding and collecting the nanofiber having the 3-D network structure of a yarn shape using a winder.

The step (a) is the step of fabricating a mixed solution in which ionic liquids and color change dyes are mixed with a polymer solution in which a polymer is dissolved in a solvent. One or two kinds of mixtures selected from the group consisting of polyperfuryl alcohol (PPFA), polymethyl methacrylate (PMMA), polyacryl copolymer, polyvinyl acetate (PVAc), polyvinylacetate copolymer, polystyrene (PS), polyvinylpyrrolidone (PVP), polystyrene copolymer, polyethylene oxide (PEO), polyethylene oxide copolymer, polycarbonate (PC), polyvinyl chloride (PVC), polypropyleneoxide copolymer, polycaprolactone, polyvinylfluoride, polyvinyllidene fluoride (poly(vinylidene fluoride) (PVDF)), polyvinyllidenefluoride copolymer, polyimide, polyacrylonitrile (PAN), 73-49 polyethylene terephthalate (PET), polypropyleneoxide (PPO), polyvinylalcohol (PVA), styrene-acrylonitrile (SAN), polycarbonate (PC), polyaniline (PANI), polypropylene (PP) and polyethylene (PE) may be used as the polymer.

The polymer configuring the electro-spinning solution may be fabricated to have a range of a weight ratio 0.1 ~ 90 wt% in each specific solvent. In the electro-spinning solution, one or two kinds of mixed solvents selected from the group consisting of deionized water, tetrahydrofuran, methanol, isopropanol, formic acid, acetonitrile, nitromethane, acetic acid, ethanol, acetone, ethylene glycol (EG), dimethyl sulfoxide (DMSO), dimethylformamide (DMF), 73-51 dimethylacetamide (DMAc) and toluene may be used as the solvent.

One or two kinds of mixtures selected from the group consisting of 1-n-butyl-3-methylimidazolium tetrafuloroborate ([C₄ₘᵢₘ] [BF₄]), 1-n-butyl-3-methylimidazolium hexafulorophosphate ([C₄ₘᵢₘ] [PF₆]), 1-n-butyl-3-methylimidazolium bis(trifluoromethylsulfonyl)imide ([C₄ₘᵢₘ] [Tf₂N]), 1-n-butyl-3-methylimidazolium bromide ([C₄ₘᵢₘ] [Br]), 1-ethyl-3-methylimidazolium hexafulorophosphate ([C₂ₘᵢₘ] [PF₆]), 1-ethyl-3-methylimidazolium bis (trifluoromethylsulfonyl) imide ([C₂ₘᵢₘ] [Tf₂N]), 1-hexyl-3-methylimidazolium tetrafuloroborate ([C₆ₘᵢₘ] [BF₄]), 1-hexyl-3-methylimidazolium hexafulorophosphate ([C₆ₘᵢₘ] [PF₆]), 1-hexyl-3-methylimidazolium bis(trifluoromethylsulfonyl)imide ([C₆ₘᵢₘ] [Tf₂N]), 1-octyl-3-methylimidazolium tetrafluoroborate ([C₈ₘᵢₘ] [BF₄]), 1-octyl-3-methylimidazolium bis (trifluoromethylsulfonyl) imide ([C₈ₘᵢₘ] [Tf₂N]), trihexyl(tetradecyl)phosphonium pyrazole ([P66614][Pyr]), trihexyl(tetradecyl)phosphonium imidazole ([P66614][Im]), trihexyl(tetradecyl)phosphonium indole ([P66614][Ind]), trihexyl(tetradecyl)phosphonium Trizole ([P66614] [Triz]), trihexyl(tetradecyl)phosphonium bentrizole ([P66614] [Bentriz]), trihexyl(tetradecyl)phosphonium tetrazole ([P66614] [Tetz]), and trihexyl(tetradecyl)phosphonium bromide ([P66614] [Br]) may be used as the ionic liquids configuring the electro-spinning solution.

According to the invention step (b) is the step of selectively dissolving the color change dyes by heating the electro-spinning solution fabricated in the step (a), at a temperature of a melting point or higher of the color change dyes. The color change dyes may be added to the polymer solution including the ionic liquids. The electro-spinning solution including the ionic liquids, in which the color change dyes have been dissociated by heating the polymer solution at a melting point or more of the color change dyes, may be prepared. If the heating temperature exceeds 150°C, the deformation or decomposition of the polymer may be caused. Accordingly, the heating temperature may be selected between 50 ~ 150°C. Color change dyes capable of being dissociated within the temperature range may be used.

In the method, the step (c) includes refining and re-crystallizing the dissociated color change dyes by quenching the electro-spinning solution fabricated in the step (b) at a temperature of 25°C or less. The color change dyes dissociated within the polymer spinning solution through the high-temperature heating process may be precipitated again into smaller particles through the quenching process, and are re-crystallized within the electro-spinning solution including the polymer having high viscosity and the ionic liquids. Accordingly, the electro-spinning solution in which fine color change dye particles have been uniformly distributed can be fabricated.

In the method, the step (d) is the step of spinning the complex polymer nanofiber in which the ionic liquids and the color change dyes have been anchored using the electro-spinning process of the electro-spinning solution, and may be the step of creating an environment in which the ionic liquids are uniformly distributed on a nanofiber surface to adsorb specific gas molecules and of maximizing the amount of the color change dye exposed to the air because they are uniformly anchored within the nanofiber and on a surface of the nanofibers. If anchoring strength of the color change dyes and the nanofiber is weak, the color change dyes may be easily desorbed on the nanofiber. If the electro-spinning process is used, the color change dyes have excellent stability because they are surrounded by the polymer of the nanofiber structurally and physically. The nanofiber is wound on the support of a wire form through the multi-electro-spinning process and the current collector, and finally composed into a yarn structure. Accordingly, the nanofiber can be used as an independent type colorimetric gas sensor.

In the method, the step (e) may include the step of winding and collecting the colorimetric gas sensor based on a composed nanofiber yarn in a thread form using a winder.

In another aspect, there is provided a gas sensor configured with a polymer nanofiber in which ionic liquids accelerating the adsorption of a specific gas and color change dyes having a varying color through a reaction with molecules of the specific gas have been anchored. The polymer nanofiber is wound on a support having a wire form to form a three-dimensional (3-D) network structure and form an independent type yarn structure.

The 3-D network structure may be a 3-D porous membrane structure in which the polymer nanofibers having a structure of a 1-D shape are randomly tangled on the support.

The ionic liquids may have high solubility for a specific gas, may have steam pressure of 10⁻⁹ ~ 10⁻¹² Pa at room temperature, may be remained on a surface of the polymer nanofiber, and may be functionalized without being evaporated even after electro-spinning.

The grain size of color change dye may have a diameter of 1 nm ~ 1 µm.

The polymer nanofiber may have a diameter of 100 nm ~ 10 µm.The yarn structure may have a diameter of 10 µm ~ 1000 µm.

The support may have a diameter of 1 ~ 5,000 µm and may have tensile strength of 50 ~ 3,000 MPa.

At least one of metal selected from a group consisting of Fe, W, Ti, Cu, Ni, Zn, and stainless steel, a natural fiber selected from a group consisting of cotton, linen, silk, wool, and a man-made fiber selected from a group consisting of nylon, polyester, acryl, polyvinylalcohol polyvinylloid, polyethylene, polypropylene, polyurethane, rayon, an acetate glass fiber, and a metal fiber may be used as the support.

The weight ratio of the ionic liquids may be 0.1 ~ 100 wt% compared to the weight of a polymer used in the polymer nanofiber.

The weight ratio of the color change dyes may have a concentration range of 0.1 ~ 400 wt% compared to the weight of a polymer used in the polymer nanofiber.

### BRIEF DESCRIPTION OF THE DRAWINGS

The accompany drawings, which are included as a part of the detailed description in order to help understanding of embodiments.

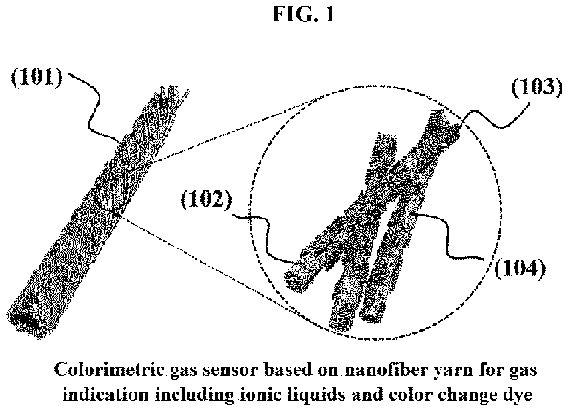

FIG. 1 is a diagram of a colorimetric gas sensor based on a complex polymer nanofiber yarn for gas indication in which ionic liquids and color change dyes have been anchored in a 1-D nanofiber structure according to an embodiment.

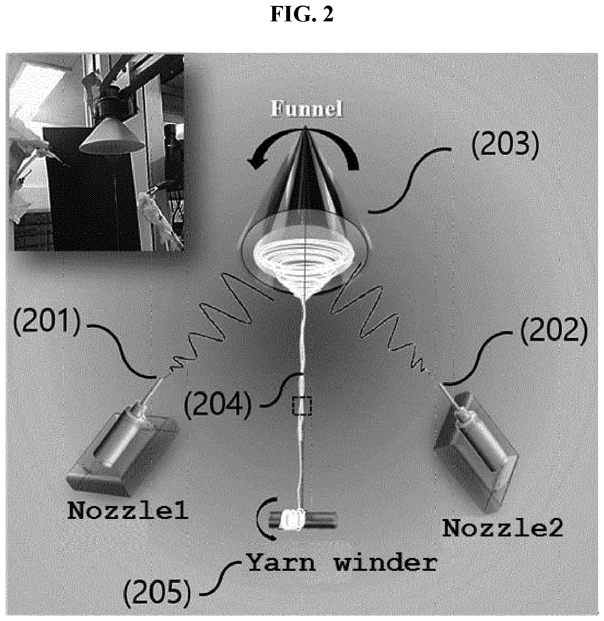

FIG. 2 illustrates a process of fabricating the colorimetric gas sensor based on a complex polymer nanofiber yarn for gas indication in which the ionic liquids and the color change dyes have been anchored in the 1-D nanofiber structure using a dual electro-spinning process according to an embodiment.

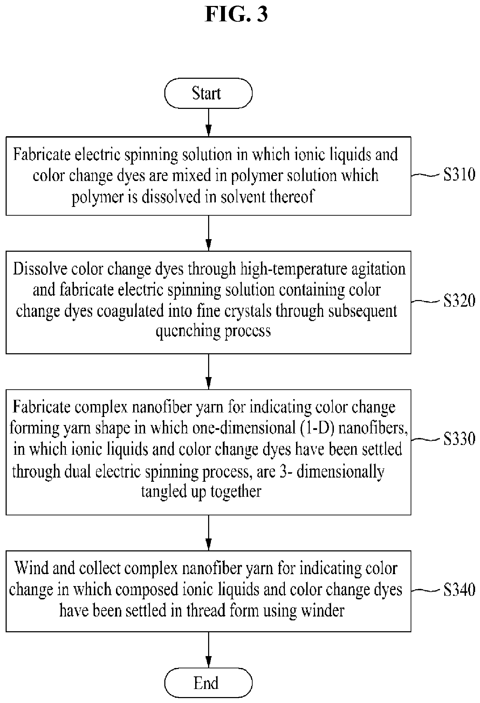

FIG. 3 is a flowchart illustrating a method of fabricating the colorimetric gas sensor based on a complex polymer nanofiber yarn for gas indication, in which the ionic liquids and the color change dyes have been anchored in the 1-D nanofiber structure using a dual electro-spinning process, and a high-temperature stirring and quenching process according to an embodiment 1.

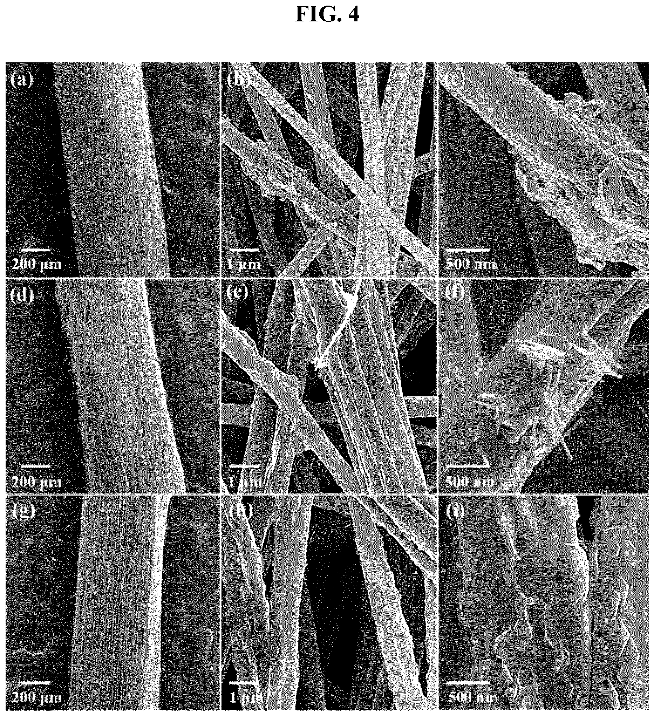

FIG. 4 illustrates photos of a colorimetric gas sensor based on a complex polymer nanofiber yarn for gas indication, in which ionic liquids (trihexyl(tetradecyl)phosphonium bromide) having (a-c) 20 wt%, (d-f) 40 wt%, (g-i) 80 wt% content and color change dyes (lead(II) acetate) having the same content (e.g., 160 wt%) compared to a polymer weight were anchored, which were photographed by a scanning electron microscope according to an embodiment.

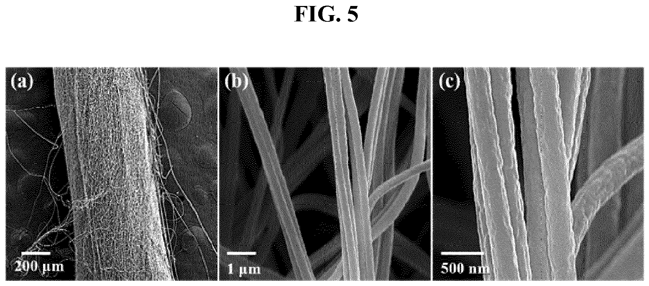

FIG. 5 illustrates photos of a colorimetric gas sensor based on a complex polymer nanofiber yarn for gas indication in which ionic liquids are not included and color change dyes (lead(II) acetate) of 160 wt% compared to a polymer weight were anchored, fabricated according to a comparative example 1, which were photographed by a scanning electron microscope.

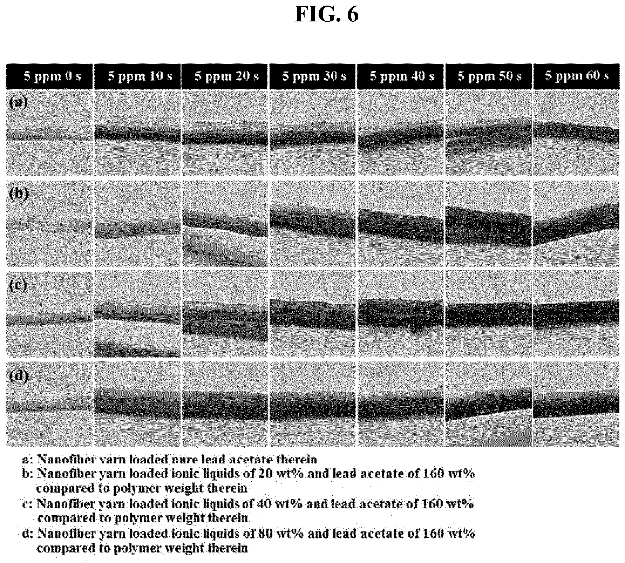

FIG. 6 illustrates photo images of color change degrees obtained by exposing, to hydrogen sulfide gas, a colorimetric gas sensor based on a complex polymer nanofiber yarn for gas indication, in which ionic liquids (trihexyl(tetradecyl)phosphonium bromide) of 20, 40, and 80 wt% and color change dyes (lead(II) acetate) of 160 wt% compared to a polymer weight were anchored, fabricated according to an embodiment 1, and a colorimetric gas sensor based on a complex polymer nanofiber yarn for gas indication, in which only color change dyes (lead(II) acetate) of 160 wt% compared to a polymer weight was anchored, fabricated according to a comparative example 1, at 5 ppm for 0, 10, 20, 30, 40, 50, and 60 seconds.

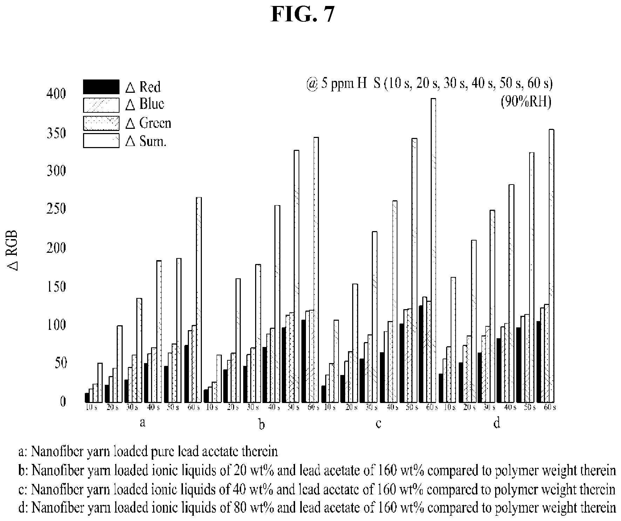

FIG. 7 is a graph quantitatively illustrating color change degrees (RGB changes) before and after the exposure of hydrogen sulfide gas for each exposure time of each sample in order to quantitatively analyze more precise color changes based on the photo images of FIG. 6.

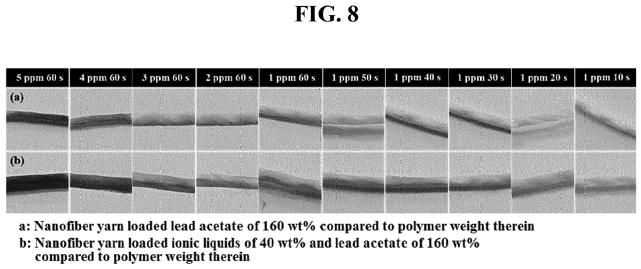

FIG. 8 illustrates photo images of color change degrees obtained if hydrogen sulfide gases of 5, 4, 3, 2, and 1 ppm are exposed for 60 seconds, and if an exposure time at 1 ppm was adjusted to 60, 50, 40, 30, 20, and 10 seconds with respect to a colorimetric gas sensor based on a complex polymer nanofiber yarn for gas indication, in which ionic liquids (trihexyl(tetradecyl)phosphonium bromide) of 40 wt% and color change dyes (lead(II) acetate) of 160 wt% compared to a polymer weight were anchored, determined to have the most excellent color change characteristics based on the results of FIGS. 6 and 7 among samples fabricated according to an embodiment 1, and a colorimetric gas sensor based on a complex polymer nanofiber yarn for gas indication, in which only color change dyes (lead(II) acetate) of 160 wt% compared to a polymer weight was anchored, fabricated according to a comparative example 1.

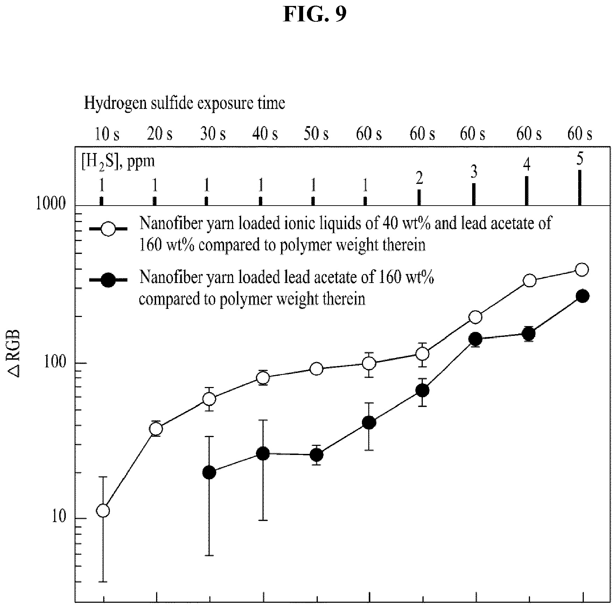

FIG. 9 is a graph quantitatively illustrating color change degrees (RGB changes) before and after the exposure to hydrogen sulfide gas for each concentration and each exposure time of each sample in order to quantitatively analyze more precise color changes based on the photo images of FIG. 8.

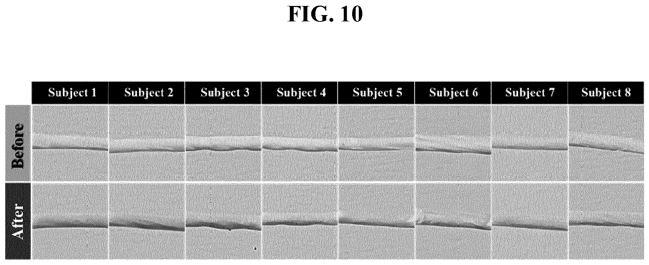

FIG. 10 illustrates photo images of color change degrees obtained before and after a colorimetric gas sensor based on a complex polymer nanofiber yarn for gas indication, in which ionic liquids (trihexyl(tetradecyl)phosphonium bromide) of 40 wt% and color change dyes (lead(II) acetate) of 160 wt% compared to a polymer weight were anchored, determined to have the most excellent color change performance among samples fabricated according to an embodiment 1, was exposed to exhaled breaths of 8 healthy persons, collected in respective Tedlar bags, and to a simulated halitosis patient exhaled breath mixed with hydrogen sulfide gas of a 1 ppm concentration for 1 minute.

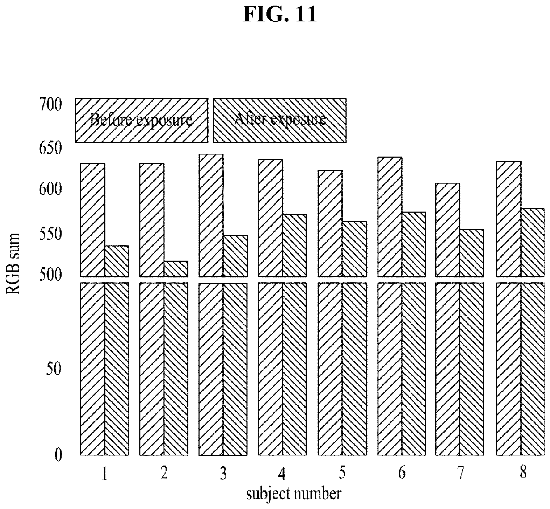

FIG. 11 is a graph quantitatively illustrating color change degrees (RGB sums) before and after the exposure to simulated halitosis breath patients' exhaled breath for each person in order to quantitatively analyze more precise color changes based on the photo images of FIG. 10.

### DETAILED DESCRIPTION

Terms, such as the first and the second, may be used to describe various elements, but the elements are not restricted by the terms. The terms are used to only distinguish one element from the other element.

Hereinafter, a colorimetric gas sensor based on a complex polymer nanofiber yarn for gas indication, including ionic liquids and color change dyes, and a method of fabricating the same, are described in detail with reference to the accompanying drawings.

In general, a colorimetric gas sensor can easily monitor and detect whether specific gas molecules are present and the concentration of specific gas molecules through the quantitative analysis of a color change degree because the specific gas molecules are absorbed by color change dyes and the color of the dyes are changed due to a chemical reaction. Furthermore, the colorimetric gas sensor has advantages in that it does not require a special electric device, can be easily reduced in size, has excellent portability, and requires a short detection time. However, since a concentration of the limit of detection of the colorimetric gas sensor is much higher than that of other gas sensors, research of a colorimetric gas sensor for improving a high concentration of the limit of detection is actively carried out.

The existing colorimetric gas sensor chiefly uses a method of simply anchoring color change dyes in a polymer by using dip-coating method in order to prevent the color change dyes from being detached out of a product during use. In the existing composition method, however, it is difficult to achieve excellent color change performance because color change dyes are rarely anchored uniformly on a color change membrane and the color change membrane does not have a sufficient specific surface area and porosity. Furthermore, an excessive amount of color change dyes needs to be used to achieve color change performance having a specific level. A colorimetric gas sensor is chiefly used as a disposable product because a color change in color change dyes is commonly irreversible. Furthermore, the colorimetric gas sensor has a danger of causing environmental pollution because heavy metal elements are included in many color change dyes.

Accordingly, in order for the colorimetric gas sensor to be widely commercialized, an ideal environment in which color change dyes cause a sharp color change through an effective and fast surface chemical reaction with a target gas even though very small amount of the color change dyes is used. Furthermore, there is a need for a colorimetric gas sensor having high sensitivity and fast response speed under the conditions. A colorimetric gas sensor for healthcare requires high sensitivity and selectivity. The colorimetric gas sensor needs to have an open structure in which the diffusion of gas is easy so that color change dyes can easily react with target gas molecules because specific gas molecules cause a color change through an adsorption and surface chemical reaction with the surface of the color change dyes. Accordingly, if a nanostructure having a high specific surface area and porosity is used as a membrane for a colorimetric gas sensor, colorimetric gas sensor sensitivity significantly improved compared to a material having a simple film form may be expected.

An example of the colorimetric gas sensor includes a hydrogen sulfide (H₂S) colorimetric gas sensor for gas indication. Hydrogen sulfide is a biomarker gas for a halitosis breath. The exhaled breath of a normal person includes hydrogen sulfide (50-80 ppb). In contrast, in the case of a halitosis patient, a hydrogen sulfide gas having a high concentration of a 1∼2 ppm level is included in the exhaled breath. In this case, lead(II) acetate may become the color change dye capable of selectively detecting the hydrogen sulfide gas. However, a hydrogen sulfide colorimetric gas sensor for gas indication may be used for industrial sites because it has a high limit of detection of 5 ppm level, which is not sensitive enough to be applied to a colorimetric gas sensor for healthcare applications.

In order to improve the sensitivity of a colorimetric gas sensor, it is necessary to maximize a surface area where color change dyes are exposed to the air and to have many reaction sites. A colorimetric gas sensor having a film form has low efficiency and sensitivity for use of color change dyes because a chemical reaction is chiefly concentrated on a surface of a film and color change dyes within the film do not participate in the reaction.

Accordingly, an embodiment provides a color change sensor based on a complex polymer nanofiber yarn for gas indication, in which ionic liquids capable of accelerating the adsorption of specific gas molecules and color change dyes having a varying color through a reaction with the specific gas molecules have been anchored, and a method of fabricating the same. The surface area where the color change dyes are exposed to a target gas can be significantly increased because the color change dyes are anchored in a nanofiber yarn-based membrane having a 3-D open pore structure. Furthermore, the diffusion of target gas can be facilitated, and the color change sensor may have much deeper color change intensity than a 2-D film type color change sensor. Furthermore, there is provided an independent type colorimetric gas sensor based on a high-performance nanofiber yarn, which can accelerate reaction characteristics with color change dyes because the adsorption property of specific gas molecules are increased by introducing ionic liquids, and a method of fabricating the colorimetric gas sensor.

According to embodiments, a nanostructure having various pores that is not a substance having such a film form can be fabricated, and a gas sensor, in which such nanostructures form a 3-D network structure and have independent type fiber forms in a thread form, can be fabricated. Such a gas sensor may be applied to environments having various curves, and may also be applied as a wearable type colorimetric gas sensor in combination with textiles.

An embodiment provides a complex polymer nanofiber (yarn-based) colorimetric gas sensor for gas indication, wherein 1-D nanofibers, each one including color change dyes causing a color change capable of being visually identified through a reaction with specific gas molecules and ionic liquids capable of selectively adsorbing a specific gas because they have high solubility for the specific gas, are tangled together to form an independent type nanofiber yarn network structure having a thread form, and a method of fabricating the colorimetric gas sensor.

There may be provided a complex polymer nanofiber, within which ionic liquids and color change dyes have been uniformly anchored and, in which the ionic liquids and the color change dyes have been uniformly anchored on a surface thereof using an electro-spinning process. The complex polymer nanofibers are wound on a support of a wire form positioned in a core through multi-electro-spinning and the rotation of a current collector, thereby being capable of fabricating a sensor having an independent type yarn structure.

In an embodiment, a nanofiber yarn is basically configured with a porous nanofiber network having a high specific surface area and excellent gas diffusion. The area, in which color change dyes are exposed to the air, can be maximized by uniformly anchoring the color change dyes in a nanofiber.

In general, content of color change dyes needs to be minimized and colorimetric gas sensor characteristics need to be maximized because most color change dyes are harmful to the environment. That is, it is necessary to create an optimal environment for increasing reactivity between specific gas molecules and color change dyes. For example, there may be introduced a substance capable of improving the surface adsorption property of specific gas molecules. If a specific gas can be selectively adsorbed, the surface chemical reaction between color change dyes and the adsorbed gas can be accelerated. Accordingly, a maximized color change characteristic can be expected even with minimum color change dyes content.

An embodiment provides a method of fabricating a colorimetric gas sensor having an independent type yarn structure of a thread form, wherein ionic liquids and color change dyes are uniformly anchored on a 1-D nanofiber using a multi-electro-spinning method and the nanofibers are wound in a bundle form. A gas sensor using such a fabrication method can provide an environment in which the reactivity of color change dyes can be improved by exposing the color change dyes to a surface of a complex nanofiber as much as possible and increasing adsorption property for specific gas molecules through ionic liquids. Accordingly, a color change characteristic having high sensitivity can be induced even with minimum content of color change dyes.

The figures describe embodiments of colorimetric gas sensors obtained by the method of the present invention.

FIG. 1 is a diagram illustrating a colorimetric gas sensor 101 based on a complex polymer nanofiber yarn according to an embodiment of the present invention.

The colorimetric gas sensor 101 based on a complex polymer nanofiber yarn has polymer nanofibers 102 wound in a 3-D network structure in a bundle form. The polymer nanofiber 102 may include color change dyes 103 having a varying change through adsorption and reactions with specific gas molecules and ionic liquids 104 which induce the adsorption of a specific gas. In this case, in the colorimetric gas sensor 101 based on a complex polymer nanofiber yarn, 1-D nanofibers tangled together randomly may be wound in a bundle form to form a nanofiber yarn structure having a thread form.

A frequency change of a wavelength performed within a visible ray area (380-780 nm) after adsorption and reactions with a specific gas, a frequency change of a wavelength from a visible ray area to an infrared area (> 780 nm) or an ultraviolet area (< 380 nm), a frequency change of a wavelength from an infrared area or ultraviolet area to a visible ray area or a change in the color change characteristic, such as color, chroma, luma or perception, due to a change in the amplitude of a wavelength may occur in the polymer nanofiber 102. The color change dyes 103 whose range of an average diameter is 1 nm ~ 1 um can be uniformly anchored without aggregation in the polymer nanofiber 102. For example, one or two kinds of mixtures selected from the group consisting of lead(II) acetate (Pb(CH₃COO)₂), iron(II) acetate (Fe(CH₃COO)₂), nickel (II) acetate (Ni(CH₃COO)₂), copper (II) acetate (Cu(CH₃COO)₂), cadmium acetate (Cd(CH₃COO)₂), cobalt(II) acetate(Co(CH₃COO)₂), manganese(II) acetate (Cu(CH₃COO)₂), bismuth (III) acetate (Co (CH₃COO)₃), silver (I) acetate(Ag(CH₃COO)), silver nitride (AgNO₃), otolidine, m-tolidine, bromophenol blue + TBAH, methyl red + TBAH, thymol blue + TBAH, fluorescein, bromocresol purple, bromophenol red, LiNO₃, 5-10-15-20- tetraphenylporphyrinatozinc (II), 5-10-15-20-tetrakis(2,4,6- trimethylphenyl)porphyrinatozinc (II) may be used as the color change dyes 103.

Furthermore, the ionic liquids 104 capable of improving the adsorption property of a specific gas by increasing the solubility of the specific gas are uniformly distributed in the polymer nanofiber 102. In this case, at least one kind selected from the group consisting of 1-n-butyl-3-methylimidazolium tetrafuloroborate ([C₄ₘᵢₘ] [BF₄]), 1-n-butyl-3-methylimidazolium hexafulorophosphate ([C₄ₘᵢₘ] [PF₆]), 1-n-butyl-3-methylimidazolium bis (trifluoromethylsulfonyl) imide ([C₄ₘᵢₘ] [Tf₂N]), 1-n-butyl-3-methylimidazolium bromide ([C₄ₘᵢₘ] [Br]), 1-ethyl-3-methylimidazolium hexafulorophosphate ([C₂ₘᵢₘ] [PF₆]), 1-ethyl-3-methylimidazolium bis(trifluoromethylsulfonyl)imide ([C₂ₘᵢₘ] [Tf₂N]), 1-hexyl-3-methylimidazolium tetrafuloroborate ([C₆ₘᵢₘ] [BF₄]), 1-hexyl-3-methylimidazolium hexafulorophosphate ([C₆ₘᵢₘ] [PF₆]), 1-hexyl-3-methylimidazolium bis (trifluoromethylsulfonyl) imide ([C₆ₘᵢₘ] [Tf₂N]), 1-octyl-3-methylimidazolium tetrafluoroborate ([C₈ₘᵢₘ] [BF₄]), 1-octyl-3-methylimidazolium bis (trifluoromethylsulfonyl) imide ([C₈ₘᵢₘ] [Tf₂N]), trihexyl(tetradecyl)phosphonium pyrazole ([P66614][Pyr]), trihexyl(tetradecyl)phosphonium imidazole ([P66614][Im]), trihexyl(tetradecyl)phosphonium indole ([P66614][Ind]), trihexyl(tetradecyl)phosphonium Trizole ([P66614][Triz]), trihexyl(tetradecyl)phosphonium bentrizole ([P66614] [Bentriz]), trihexyl(tetradecyl)phosphonium tetrazole ([P66614][Tetz]), trihexyl(tetradecyl)phosphonium bromide ([P66614] [Br]), for example, may be used as the ionic liquids 104.

The diameter of the polymer nanofiber 102 produced through an electro-spinning process may be included in a range of 100 nm ~ 10 um. The color change dyes 103 of the listed acetate series are dyes capable of showing a specific color change through a reaction with a hydrogen sulfide gas, in particular. In addition to the listed dyes, color change dyes that cause a color change characteristic through a selective reaction and combination with a specific gas may be unlimitedly used in a combination with the nanofiber 102 including the ionic liquids 104.

FIG. 2 is a diagram of a device for a nanofiber yarn composition through multi-electro-spinning used in an embodiment 1 of the present invention.

An electro-spinning solution including the ionic liquids and color change dyes may be discharged from the needles of two different syringes 201 and 202 through an electro-spinning process, and may be discharged in the form of a 1-D complex polymer nanofiber membrane. A strong electric field from a high voltage application device is applied to the needles of the syringes 201 and 202. The discharged electro-spinning solution is spun on a current collector 203. In this case, complex polymer nanofibers spun from the two syringes 201 and 202 may be wound on a surface of a thread 204 at a core by the rotation of the current collector 203 in a yarn form. The wound complex polymer nanofiber yarn may be wound by a winder 205 and collected in a thread form.

One or two kinds of mixed solvents selected from the group consisting of deionized water, tetrahydrofuran, methanol, isopropanol, formic acid, acetonitrile, nitromethane, acetic acid, ethanol, acetone, ethylene glycol (EG), dimethyl sulfoxide (DMSO), dimethylformamide (DMF), dimethylacetamide (DMAc) and toluene(toluene), for example, may be used as a solvent used when the polymer spinning solution is fabricated.

Furthermore, one or two kinds of mixtures selected from the group consisting of polyperfuryl alcohol (PPFA), polymethyl methacrylate (PMMA), polyacryl copolymer, polyvinyl acetate (PVAc), polyvinylacetate copolymer, polystyrene (PS), polyvinylpyrrolidone (PVP), polystyrene copolymer, polyethylene oxide (PEO), polyethylene oxide copolymer, polycarbonate (PC), polyvinyl chloride (PVC), polypropyleneoxide copolymer, polycaprolactone, polyvinylfluoride, polyvinyllidenefluoride (poly(vinylidene fluoride) (PVDF)), polyvinyllidenefluoride copolymer, polyimide, polyacrylonitrile (PAN), 73-49 polyethylene terephthalate (PET), polypropyleneoxide (PPO), polyvinylalcohol (PVA), styrene-acrylonitrile (SAN), polycarbonate (PC), polyaniline (PANI), polypropylene (PP) and polyethylene (PE), for example, may be used as the polymer.

In order to achieve the viscosity suitable for performing electro-spinning, the polymer configuring the electro-spinning solution may be fabricated in a concentration range of a weight ratio 0.1 ~ 90 wt% in a specific solvent. The weight ratios of the ionic liquids and the color change dyes may have concentration ranges of 0.1 ~ 100 wt% and 0.1 ~ 400 wt%, respectively, compared to a polymer matrix.

The ionic liquids and color change dyes may be distributed and used in a polymer solution, in which the polymer is dissolved in the solvent, or a polymer solution refined and re-crystallized from color change dyes because a mixed electro-spinning solution experiences a high-temperature stirring and quenching process may be used for the ionic liquids and color change dyes.

Furthermore, the high-temperature stirring and quenching process of the present invention includes a process of dissolving color change dyes in a solvent by stirring a mixed electro-spinning solution in which the color change dyes, ionic liquids and a polymer have been dissolved in the solvent at a melting point or higher of the color change dyes, and quenching the mixed electro-spinning solution at temperature of 25°C or lower after the stirring for a sufficient time.

In the electro-spinning process, the amount of discharge for discharging the electro-spinning solution may have a range of 0.1 ~ 100 µl/min. This may be properly selected depending on the viscosity of a spinning solution. The diameter of the nanofiber that is finally composed may be controlled by adjusting a ratio of a concentration of the spinning solution and an injection speed. A voltage of a 1 ~ 30 kV range may be applied between the nozzle and the current collector. The distance between the nozzle and the current collector may be selected in a range of 1 ~ 30 cm. The rotation velocity of the current collector at which the nanofiber yarn is composed may be selected in a range of 10 ~ 500 rpm, and the composed nanofiber yarn may be collected at the velocity of 1 ~ 400 mm/min through the winder.

If the colorimetric gas sensor based on a complex polymer nanofiber yarn, in which the ionic liquids and the color change dyes have been anchored, is exposed to a specific gas, the adsorption of the molecules of the specific gas on a surface of each fiber is accelerated due to an ionic liquid effect. A color change may appear due to a surface chemical reaction between the adsorbed gas molecules and the color change dyes. The colorimetric gas sensor based on a complex polymer nanofiber yarn for gas indication detects environment noxious gases (e.g., H₂S, SOₓ, NOₓ, COₓ) and a biomarker gas (e.g., CH₃COCH₃, C₂H₅OH, C₆H₅CH₃) included in the exhaled breath of a person.

FIG. 3 is a flowchart illustrating a method of fabricating a colorimetric gas sensor based on a complex polymer nanofiber yarn, in which ionic liquids and color change dyes have been anchored through a multi-electro-spinning process according to an embodiment of the present invention.

The fabrication method may include the step 301 of fabricating an electro-spinning solution, in which ionic liquids and color change dyes are mixed in a polymer solution having a polymer dissolved in a solvent, the step 302 of dissolving the color change dyes through high-temperature stirring at a melting point or more of the color change dyes and fabricating a complex electro-spinning solution containing color change dyes re-crystallized into fine crystals through a subsequent quenching process, the step 303 of composing a color change sensor based on a complex polymer nanofiber yarn, in which the ionic liquids and color change dyes have been anchored using a multi-electro-spinning process, and the step 304 of winding and collecting the color change sensor based on a composed complex polymer nanofiber yarn in a thread form.

The present invention is described below specifically in connection with embodiments and comparative examples. The embodiments and the comparative examples are merely from describing the present invention, and the present invention is not limited to them.

### Embodiment 1: Fabrication of a colorimetric gas sensor based on a complex polymer nanofiber yarn for gas indication, including ionic liquids and color change dyes, using a dual electro-spinning process including ionic liquids and color change dyes

First, in order to prepare an electro-spinning solution to be injected into a syringe, polyacrylonitrile (PAN) 0.75 g having molecular weight of 130,000 g/mol is dissolved in dimethylformamide (DMF) of 9 ml. Additionally, ionic liquids (trihexyl(tetradecyl)phosphonium bromide) of 20, 40, and 80 wt% compared to a polymer weight ratio and lead(II) acetate of 160 wt% compared to the polymer weight ratio are included in a polymer/solvent complex solution, and a spinning solution is fabricated by stirring the polymer/solvent complex solution at 500 rpm for 12 hours at a high temperature of 85°C. The color change dyes (lead(II) acetate) are dissolved through sufficient stirring at high temperature. The refinement and re-crystallization of the dissociated color change dyes (lead(II) acetate) are induced by quenching an electro-spinning solution in which the polymer (PAN) and the ionic liquids (trihexyl(tetradecyl)phosphonium bromide) have been dissolved in the solvent (DMF) at a temperature of about 25°C. The spinning solution fabricated through the high temperature stirring and quenching process is contained in each of two different syringes (e.g., Henke-Sass Wolf, 12 ml NORM-JECT). The syringes are connected to syringe pumps. The electro-spinning solutions are pushed at a discharge velocity of 10 µl/min. Electro-spinning is performed by applying a voltage of 12 kV between a nozzle (23 gauge) used in a spinning process and a current collector for collecting a nanofiber. In this case, the discharged nanofiber is collected on the current collector of a hopper form that rotates at about 300 rpm. The nanofiber is wound on a support of a wire form at a velocity of 100 mm/min, and is wound and collected on a winder in a yarn structure having a thread form.

FIG. 4 illustrates photos of a scanning electron microscope with respect to a complex polymer nanofiber yarn for gas indication, which was fabricated according to an embodiment 1 of the present invention and in which ionic liquids (trihexyl(tetradecyl)phosphonium bromide) of (a-c) 20 wt%, (d-f) 40 wt%, and (g-i) 80 wt% compared to a polymer weight were included and color change dyes (lead(II) acetate) having the same content (e.g., 160 wt%) were anchored.

From the photos of the scanning electron microscope for the complex polymer nanofiber yarn fabricated through electro-spinning, that includes the ionic liquids and the color change dyes, it can be seen that the color change dyes (lead (II) acetate) having a size range of 1 um or less are well anchored in a polymer nanofiber having a diameter of about 400 ~ 600 nm. In particular, the diameter of the nanofiber tends to increase on average as the content of the ionic liquids (trihexyl(tetradecyl)phosphonium bromide) increases. The reason for this is that the diameter of the nanofiber is proportional to the viscosity of the electro-spinning solution and the viscosity of the ionic liquids (trihexyl(tetradecyl)phosphonium bromide) is very high. All nanofiber yarns fabricated regardless of the content of the ionic liquids (trihexyl(tetradecyl)phosphonium bromide) have the same size of about 600 um (see (a), (d) and (g) of FIG. 4) .

### Comparative example 1. Fabrication of a colorimetric gas sensor based on a complex polymer nanofiber yarn for gas indication, that includes color change dyes using a dual electro-spinning process; including only the color change dyes other than ionic liquids

In the comparative example 1 compared to the embodiment 1, a color change sensor based on a complex polymer nanofiber yarn for gas indication, in which only color change dyes (lead(II) acetate) have been anchored on a polymer nanofiber by electrically spinning an electro-spinning solution, including the color change dyes having the same amount as those of the embodiment 1, but without including ionic liquids, may be fabricated. Specifically, first, polyacrylonitrile (PAN) 0.75 g having a molecular weight of 130,000 g/mol is dissolved in dimethylformamide (DMF) of 9 ml. Additionally, lead (II) acetate of 160 wt% compared to a polymer weight ratio is included in a polymer/solvent complex solution, and a spinning solution is fabricated by stirring the polymer/solvent complex solution at 500 rpm for 12 hours at a high temperature of 85°C. The color change dyes (lead(II) acetate) are dissociated through sufficient stirring. The refinement and re-crystallization of the dissociated color change dyes (lead(II) acetate) are induced by quenching an electro-spinning solution in which the polymer (PAN) has been dissolved in the solvent (DMF) at a temperature of about 25°C. The spinning solution fabricated through the high-temperature stirring and quenching process is contained in each of two different syringes (e.g., Henke-Sass Wolf, 12 ml NORM-JECT) . The syringe is connected to a syringe pump. The electro-spinning solution is pushed at a discharge velocity of 10 µl/min. Electro-spinning is performed by applying a voltage of 12 kV between a nozzle (or needle, 23 gauge) used for the spinning process and a current collector for collecting a nanofiber. In this case, the discharged nanofiber is collected on the current collector of a hopper form that rotates at about 300 rpm. The collected nanofiber is wound on a support of a wire form at a velocity of 100 mm/min and wound and collected on a winder in a yarn structure having a thread form.

FIG. 5 illustrates photos of a scanning electron microscope for a complex polymer nanofiber yarn for gas indication, which was fabricated according to the comparative example 1 of the present invention and in which color change dyes (lead (II) acetate) of 160 wt% compared to a polymer weight were included.

It may be seen that the diameter of a nanofiber yarn is about 600 µm level and is similar to the case where the ionic liquids are included (see (a) of FIG. 5), but the diameter of a composed nanofiber is a 200 nm level and is much thinner than that of the case where the ionic liquids are included (about 500 nm) because the ionic liquids having very high viscosity are not included and thus the viscosity of an electro-spinning solution is relatively lower than the sample fabricated according to an embodiment 1. Furthermore, it can be seen that the color change dyes have been unevenly anchored on the surface of the nanofiber as in the embodiment 1 (see (b) and (c) of FIG. 5).

### Experimental example 1. Evaluation of hydrogen sulfide gas detection color change characteristics using the colorimetric gas sensor based on a complex polymer nanofiber yarn for gas indication, including ionic liquids and color change dyes composed according to the embodiment 1, and the colorimetric gas sensor based on a complex polymer nanofiber yarn for gas indication, including only the color change dyes composed according to the comparative example 1

Color change characteristics for a hydrogen sulfide gas are evaluated by directly exposing the color change sensors based on a nanofiber yarn obtained according to the embodiment 1 and comparative example 1, to the hydrogen sulfide gas, while controlling the concentration and exposure time of the hydrogen sulfide gas in a high-humidity environment having relative humidity of 80% which is a similar condition as the exhaled breath of human.

FIG. 6 illustrates photo images of color change degrees obtained by directly exposing, to a hydrogen sulfide gas of 5 ppm, the colorimetric gas sensor based on a nanofiber yarn, in which ionic liquids (trihexyl(tetradecyl)phosphonium bromide) of 20, 40, and 80 wt% compared to a polymer weight are included and color change dyes (lead (II) acetate) of 160 wt% compared to the polymer weight were anchored, fabricated according to the embodiment 1, and the colorimetric gas sensor based on a nanofiber yarn, in which only color change dyes (lead(II) acetate) of 160 wt% compared to a polymer weight was anchored, fabricated according to the comparative example 1, under conditions of 0, 10, 20, 30, 40, 50, and 60 seconds.

FIG. 6 illustrates photo images of color change degrees obtained by direct exposure to hydrogen sulfide gas of 5 ppm, of the colorimetric gas sensor based on a nanofiber yarn in which ionic liquids (trihexyl(tetradecyl)phosphonium bromide) of 20, 40, and 80 wt% compared to the polymer weight are included and color change dyes (lead(II) acetate) of 160 wt% compared to the polymer weight were anchored, fabricated according to the embodiment 1, and the colorimetric gas sensor based on a nanofiber yarn in which only color change dyes (lead (II) acetate) of 160 wt% compared to a polymer weight was anchored, fabricated according to the comparative example 1, under exposure conditions of 0, 10, 20, 30, 40, 50, and 60 seconds.

From the photo images of FIG. 6, it can be seen that the color change degree gradually grows greater as the exposure time is increased for the hydrogen sulfide concentration of 5 ppm. Furthermore, it may be seen that in the case of the sensor including the ionic liquids (trihexyl(tetradecyl)phosphonium bromide), a color change degree is much greater compared to the sensor not including the ionic liquids (trihexyl(tetradecyl)phosphonium bromide). The reason for this is that the ionic liquids (trihexyl(tetradecyl)phosphonium bromide) may accelerate the color change through a surface chemical reaction with the color change dyes (lead(II) acetate) anchored on the surface of the nanofiber yarn by improving the adsorption property of hydrogen sulfide gas as the ionic liquids have high solubility for the hydrogen sulfide gas. However, if the content of the ionic liquids (trihexyl(tetradecyl)phosphonium bromide) increases too much, color change degree is decreased because a color change becomes light-brown not thick-brown due to the unique color of the ionic liquids (trihexyl(tetradecyl)phosphonium bromide). Furthermore, if the content of the ionic liquids (trihexyl(tetradecyl)phosphonium bromide) increases too much, electro-spinning is not smoothly performed because the viscosity of the electro-spinning solution increases too much. It is seen that the ideal content of the ionic liquids (trihexyl(tetradecyl)phosphonium bromide) is present.

FIG. 7 is a graph quantitatively illustrating color change degrees (RGB changes) before and after the exposure to 5 ppm hydrogen sulfide for 0, 10, 20, 30, 40, 50, and 60 seconds as in the photo images of FIG. 6, for the purpose of clearer color change quantitative analysis of the color change images illustrated in FIG. 6. It may be seen that for the sensors including the ionic liquids (trihexyl(tetradecyl)phosphonium bromide) of 20, 40, and 80 wt%, respectively, compared to the polymer weight, the sum of RGB changes is increased because R, G, and B values are increased compared to the sensor not including the ionic liquids (trihexyl(tetradecyl)phosphonium bromide). Accordingly, it may be quantitatively seen that color change degree becomes greater if the ionic liquids (trihexyl(tetradecyl)phosphonium bromide) are included. Furthermore, as in the photo images of FIG. 6, it may be seen that the greatest color change degree appears if the ionic liquids (trihexyl(tetradecyl)phosphonium bromide) having optimal content (40 wt%) are included, through quantitative analysis.

FIG. 8 illustrates photo images of color change degrees obtained if the colorimetric gas sensor based on a nanofiber yarn includes ionic liquids (trihexyl(tetradecyl)phosphonium bromide) of 40 wt% and color change dyes (lead (II) acetate) of 160 wt% compared to the polymer weight, which is determined to have the most excellent color change characteristic based on the results of FIGS. 6 and 7, and those composed through the embodiment 1 were exposed to hydrogen sulfide gases of 5, 4, 3, 2 and 1 ppm for 60 seconds and if the colorimetric gas sensor based on a nanofiber yarn including only color change dyes (lead(II) acetate) of 160 wt% compared to the polymer weight, those composed through the comparative example 1 were exposed to a hydrogen sulfide gas of 1 ppm for 60, 50, 40, 30, 20, and 10 seconds.

From the photo images of FIG. 8, it may be seen that a color change degree grows greater as the hydrogen sulfide concentration increases from 1 to 5 ppm. If the exposure time was adjusted to 60, 50, 40, 30, 20, and 10 seconds at 1 ppm hydrogen sulfide concentration, it can be seen that in the color change sensors based on the nanofiber yarn including the ionic liquids (trihexyl(tetradecyl)phosphonium bromide) of 40 wt% and the color change dyes (lead (II) acetate) of 160 wt% compared to the polymer weight, color change characteristics are much excellent compared to the color change sensor based on the nanofiber yarn including only the color change dyes (lead(II) acetate) of 160 wt% compared to the polymer weight without the ionic liquids (trihexyl(tetradecyl)phosphonium bromide). If the sample not including the ionic liquids (trihexyl(tetradecyl)phosphonium bromide) was exposed to hydrogen sulfide of 1 ppm for 40 seconds or less, whether a color change is present or not is rarely determined on the photo. However, it can be seen that in the sample including the ionic liquids (trihexyl(tetradecyl)phosphonium bromide) and the sample exposed to hydrogen sulfide of 1 ppm for 20 seconds, whether a color change is present or not can be determined with the naked eye and the color change is thus excellent.

FIG. 9 is a graph quantitatively illustrating color change degrees (RGB changes) before and after exposure to hydrogen sulfide of 1 ppm for 60, 50, 40, 30, 20, and 10 seconds as in the photo images of FIG. 8. Exposure to hydrogen sulfide of 5, 4, 3, 2, and 1 ppm for 60 seconds was done for the purpose of clearer color change quantitative analysis of the color change images is illustrated in FIG. 8. As in the photo images of FIG. 8, a low level degree of color change can be seen for the case of the sensor not including the ionic liquids (trihexyl(tetradecyl)phosphonium bromide), compared to the case of the sensor including the ionic liquids (trihexyl(tetradecyl)phosphonium bromide) even through quantitative analysis. It could be seen that in the case of the sensor not including the ionic liquids (trihexyl(tetradecyl)phosphonium bromide), quantitative analysis of the color change was impossible when the sensor was exposed to hydrogen sulfide of 1 ppm for 30 seconds or less. In contrast, it could be quantitatively seen that the sensor including the ionic liquids (trihexyl(tetradecyl)phosphonium bromide) had a color change degree of at least 10 even when it was exposed to hydrogen sulfide of 1 ppm for 10 seconds. Accordingly, an excellent catalyst characteristics for the detection of hydrogen sulfide of the ionic liquids (trihexyl(tetradecyl)phosphonium bromide) can be proved.

### Experimental example 2. Evaluation of a color change characteristics for diagnosing of a simulated halitosis breath patient using the colorimetric gas sensor based on a complex polymer nanofiber yarn for gas indication including ionic liquids and color change dyes, composed through the embodiment 1

In order to evaluate the color change characteristics of the sensor to the hydrogen sulfide gas, that is known as a biomarker gas for a halitosis breath patient, a mixed gas (i.e., simulated halitosis breath patient exhaled breath) of the exhaled breath of an actual person and the hydrogen sulfide gas are fabricated. The color change characteristic is evaluated by directly exposing the color change sensors based on the nanofiber yarn including ionic liquids and color change dyes, obtained according to the embodiment 1, to the mixed gas.

After the exhaled breaths of 8 healthy persons are collected through Tedlar bags, each of the collected exhaled breaths is mixed with a hydrogen sulfide gas of a 1 ppm level. A color change degree is evaluated by directly exposing a color change sensor based on a nanofiber yarn including ionic liquids (trihexyl(tetradecyl)phosphonium bromide) of 40 wt% and color change dyes (lead(II) acetate) of 160 wt% compared to the polymer weight, which is determined to have the most excellent color change performance among the samples composed through the embodiment 1, to the mixed gas.

FIG. 10 illustrates photo images before and after simulated halitosis breath patient exhaled breaths, fabricated by mixing exhaled breaths of 8 healthy persons and hydrogen sulfide of 1 ppm, were exposed to the color change sensor based on the nanofiber yarn, including the ionic liquids (trihexyl(tetradecyl)phosphonium bromide) of 40 wt% and the color change dyes (lead(II) acetate) of 160 wt% compared to the polymer weight.

From the photos, it can be seen that the nanofiber yarn is white color before the exposure, but is brown color after the exposure of the simulated halitosis breath for 1 minute.

FIG. 11 is a graphical diagram of color change degrees (RGB changes) before and after the exposure of the simulated halitosis patient exhaled breath as in FIG. 10, for the purpose of clearer color change quantitative analysis of the color change images illustrated in FIG. 10. It can be seen that a color change occurs through a reduction of a quantitative value of a total RGB value after the exposure of the simulated halitosis patient exhaled breaths compared to the case where the simulated halitosis patient exhaled breaths were exposed. In this case, each person is different in a quantitative value of a color change because a concentration of hydrogen sulfide included in each of the 8 person exhaled breaths may be different. However, it can be seen that the color change sensor can be applied as a color change sensor capable of rapid detecting of even small amount of hydrogen sulfide gas through simulated halitosis patient exhaled breath analysis of a gas mixture of 1 ppm hydrogen sulfide gas and an exhaled breath.

Sensor characteristics of a gas sensor sensing material can be checked by taking a biomarker gas as an example through the experimental example.

By forming a colorimetric gas sensor membrane based on a nanofiber yarn for gas indication including both ionic liquids and color change dyes, the colorimetric gas sensor based on a nanofiber yarn for gas indication, in which 1) the ionic liquids having high solubility for a specific gas selectively adsorbs specific gas molecules, 2) in which it has excellent color change performance through minimum amount of color change dyes by increasing reactivity between adsorbed gas molecules and color change dyes, and 3) in which the nanofiber forms a 3-D network structure and forms an independent yarn type structure, can be fabricated.

The colorimetric gas sensor based on a complex polymer nanofiber yarn for gas indication, including ionic liquids and color change dyes in the 1-D nanofiber structure fabricated using an electro-spinning process, can have high surface area and porosity compared to the existing colorimetric gas sensor of test paper for gas detection. Accordingly, the gas sensor according to the embodiment can detect environment noxious gases (e.g., H₂S, SOₓ, NOₓ or COₓ) and biomarker gases (e.g., CH₃COCH₃, C₂H₅OH or C₆H₅CH₃) included in a person's exhaled breath, with high sensitivity and high speed as it generates a color change within several tens of seconds even in the exposure to a low concentration of the gas at 1 ppm level. Furthermore, the gas sensor can be used for curved applications and fabricated in a cloth form through weaving using the structural characteristic of the independent yarn type structure.

The ionic liquids according to the embodiment can improve the adsorption property of a gas because it has high solubility for a specific gas, and can significantly improve color change sensitivity by accelerating the reaction with color change dyes. More importantly, the yarn can be easily processed, applied to surfaces having various curves, and combined with clothes through weaving because the nanofiber can be fabricated in an independent yarn type structure through a multi-electro-spinning process.

Furthermore, mass-production is easy because electro-spinning, having relatively low manufacturing cost, is used. The color change sensor according to the embodiment can generate a color change within several tens of seconds to the gas of 1 ppm level because it provides high specific surface area and porosity compared to the conventional test paper for gas detection. Therefore, the gas sensor according to the embodiment can be used for healthcare products for detecting environment noxious gases and a biomarker gas included in a person's exhaled breath.

The ionic liquids according to the embodiment can accelerate the surface chemical reaction between specific gas molecules and color change dyes by inducing the adsorption of the specific gas molecules. There can be provided the colorimetric gas sensor based on a nanofiber yarn for gas indication, in which ionic liquids and color change dyes have been functionalized and which has excellent color change performance with the least amount of dyes due to the gas molecule adsorption acceleration effect of the ionic liquids and the effective exposure of the color change dyes anchored on the high-density and high surface area nanofiber yarn scaffold.

## Claims

1. A method of fabricating a gas sensor, comprising steps of:
(a) fabricating a mixed solution which the ionic liquids and color change dyes are mixed with the polymer solution which a polymer is dissolved in a solvent;
(b) dissolving the ionic liquids and the color change dyes within the mixed solution through high-temperature stirring process, which high-temperature is at a temperature of a melting point or higher of the color change dyes;
(c) fabricating an electro-spinning solution containing dyes recrystallized into fine crystals through quenching process of the mixed solution which the ionic liquids and the color change dyes have been dissolved;
(d) fabricating a one-dimensional (1-D) polymer nanofiber in which the ionic liquids and the color change dyes have been anchored using a dual electro-spinning process and fabricating the 1-D polymer nanofiber into a nanofiber having a 3-D network structure of a yarn shape; and
(e) winding and collecting the nanofiber with the 3-D network structure of a yarn shape using a winder.

2. The method of claim 1, wherein the 3-D network structure is a 3-D porous membrane structure in which the polymer nanofibers having a structure of a 1-D shape are randomly tangled on the support.

3. The method of claim 1, wherein:
the ionic liquids have high solubility for the specific gas, and
the ionic liquids have steam pressure of 10⁻⁹ ~ 10⁻¹² Pa at room temperature and are left on the surface of the polymer nanofiber and are functionalized without evaporating even after electro-spinning.

4. The method of claim 1, wherein at least one of the ionic liquids are from a group consisting of 1-n-butyl-3-methylimidazolium tetrafuloroborate ([C4mim] [BF4]), 1-n-butyl-3-methylimidazolium hexafulorophosphate ([C4mim] [PF6]), 1-n-butyl-3-methylimidazolium bis(trifluoromethylsulfonyl)imide ([C4mim] [Tf2N]), 1-n-butyl-3-methylimidazolium bromide ([C4mim] [Br]), 1-ethyl-3-methylimidazolium hexafulorophosphate ([C2mim] [PF6]), 1-ethyl-3-methylimidazolium bis(trifluoromethylsulfonyl)imide ([C2mim] [Tf2N]), 1-hexyl-3-methylimidazolium tetrafuloroborate ([C6mim] [BF4]), 1-hexyl-3-methylimidazolium hexafulorophosphate ([C6mim] [PF6]), 1-hexyl-3-methylimidazolium bis(trifluoromethylsulfonyl)imide ([C6mim][Tf2N]), 1-octyl-3-methylimidazolium tetrafluoroborate ([C8mim] [BF4]), 1-octyl-3-methylimidazolium bis(trifluoromethylsulfonyl)imide ([C8mim] [Tf2N]), trihexyl(tetradecyl)phosphonium pyrazole ([P66614][Pyr]), trihexyl(tetradecyl)phosphonium imidazole ([P66614][lm]), trihexyl(tetradecyl)phosphonium indole ([P66614][Ind]), trihexyl(tetradecyl)phosphonium Trizole ([P66614][Triz]), trihexyl(tetradecyl)phosphonium bentrizole ([P66614][Bentriz]), trihexyl(tetradecyl)phosphonium tetrazole ([P66614][Tetz]), and trihexyl(tetradecyl)phosphonium bromide ([P66614][Br]).

5. The method of claim 1, wherein the color change dyes comprise a substance having at least one characteristic change of color, chroma, luma, and perception attributable to a frequency change of a wavelength within visible range, from visible range to infrared or ultraviolet range, from infrared or ultraviolet range to visible range, or an intensity change of a wavelength upon reaction with the molecules of the specific gas.

6. The method of claim 1, wherein at least one of the color change dyes are selected from a group consisting of lead(II) acetate(Pb(CH3COO)2), iron(II) acetate(Fe(CH3COO)2), nickel(II) acetate(Ni(CH3COO)2), copper(II) acetate(Cu(CH3COO)2), cadmium acetate(Cd(CH3COO)2), cobalt(II) acetate(Co(CH3COO)2), manganese(II) acetate (Cu(CH3COO)2), bismuth(III) acetate(Co(CH3COO)3), silver(I) acetate(Ag(CH3COO)), silver nitride (AgNO3), otolidine, m-tolidine, bromophenol blue + TBAH, methyl red + TBAH, thymol blue + TBAH, fluorescein, bromocresol purple, bromophenol red, LiNO3, 5-10-15-20- tetraphenylporphyrinatozinc (II), and 5-10-15-20-tetrakis(2,4,6-trimethylphenyl)porphyrinatozinc (II).

7. The method of claim 1, wherein the color change dyes have a diameter of 1 nm ~ 1 µm.

8. The method of claim 1, wherein the polymer nanofiber is formed by electric-spinning a polymer solution comprising the ionic liquids and the color change dyes.

9. The method of claim 1, wherein:
the polymer nanofiber has a diameter of 100 nm ~ 10 µm,
and
the yarn structure has a diameter of 10 µm ~ 1000 µm.

10. The method of claim 1, wherein the support has diameter of 1 ~ 5,000 µm and has tensile strength of 50 ~ 3,000 MPa.

11. The method of claim 1, wherein the support comprises at least one of the metals selected from a group consisting Fe, W, Ti, Cu, Ni, Zn, and stainless steel, a natural fiber selected from a group consisting cotton, linen, silk, and wool, and a man-made fiber selected from a group consisting of nylon, polyester, acryl, polyvinylalcohol polyvinylloid, polyethylene, polypropylene, polyurethane, rayon, an acetate glass fiber, and a metal fiber.

12. The method of claim 1, wherein a weight ratio of the ionic liquids is 0.1 wt% ~ 100 wt% compared to the weight of the polymer used in the polymer nanofiber.

13. The method of claim 1, wherein the weight ratio of the color change dyes has a concentration range of 0.1 wt% ~ 400 wt% compared to the weight of the polymer used in the polymer nanofiber.

14. The method of claim 1, wherein a polymer configuring the polymer nanofiber comprises at least one selected from a group consisting of polyperfuryl alcohol (PPFA), polymethyl methacrylate (PMMA), polyacryl copolymer, polyvinyl acetate (PVAc), polyvinylacetate copolymer, polystyrene (PS), polyvinylpyrrolidone (PVP), polystyrene copolymer, polyethylene oxide (PEO), polyethylene oxide copolymer, polycarbonate (PC), polyvinyl chloride (PVC), polypropyleneoxide copolymer, polycaprolactone, polyvinylfluoride, polyvinyllidenefluoride (poly(vinylidene fluoride) (PVDF)), polyvinyllidenefluoride copolymer, polyimide, polyacrylonitrile (PAN), 73-49 polyethylene terephthalate (PET), polypropyleneoxide (PPO), polyvinylalcohol (PVA), styrene-acrylonitrile (SAN), polycarbonate (PC), polyaniline (PANI), polypropylene (PP), and polyethylene (PE).

## Patentansprüche

1. Verfahren zur Herstellung eines Gassensors, das die folgenden Schritte umfasst:
(a) Herstellen einer gemischten Lösung, in der die ionischen Flüssigkeiten und Farbwechsel-Farbstoffe mit der Polymerlösung gemischt werden, in der ein Polymer in einem Lösungsmittel gelöst ist;
(b) Auflösen der ionischen Flüssigkeiten und der Farbwechsel-Farbstoffe in der gemischten Lösung durch Rühren bei hoher Temperatur, wobei die hohe Temperatur bei einer Temperatur liegt, die dem Schmelzpunkt der Farbwechsel-Farbstoffe entspricht oder höher ist;
(c) Herstellen einer Elel<trospinnlösung, die zu feinen I<ristallen rekristallisierte Farbstoffe enthält, durch Abschrecken der gemischten Lösung, in der die ionischen Flüssigkeiten und die Farbwechsel-Farbstoffe gelöst wurden;
(d) Herstellen einer eindimensionalen (1-D) Polymer-Nanofaser, in der die ionischen Flüssigkeiten und die Farbwechsel-Farbstoffe verankert wurden, unter Verwendung eines dualen Elel<trospinnverfahrens und Herstellen der 1-D-Polymer-Nanofaser zu einer Nanofaser mit einer 3-D-Netzwerl<strul<tur in Garnform; und
(e) Aufwickeln und Sammeln der Nanofaser mit der 3-D-Netzstrul<tur in Garnform mit einem Wickler.

2. Verfahren nach Anspruch 1, wobei die 3-D-Netzwerl<strul<tur eine poröse 3-D-Membranstruktur ist, in der die Polymer-Nanofasern mit einer Struktur einer 1-D-Form zufällig auf dem Träger verl<näuelt sind.

3. Das Verfahren nach Anspruch 1, wobei:
die ionischen Flüssigkeiten eine hohe Löslichkeit für das jeweilige Gas aufweisen, und
die ionischen Flüssigkeiten bei Raumtemperatur einen Dampfdruck von 10⁻⁹ ~ 10⁻¹² Pa haben und auf der Oberfläche der Polymer-Nanofaser verbleiben und auch nach dem Elektrospinnen ohne Verdampfung funktionalisiert werden.

4. Verfahren nach Anspruch 1, wobei mindestens eine der ionischen Flüssigkeiten aus einer Gruppe bestehend aus 1-n-Butyl-3-methylimidazoliumtetrafuloroborat ([C4mim] [BF4]), 1-n-Butyl-3-methylimidazoliumhexafulorophosphat ([C4mim] [PF6]), 1-n-Butyl-3-methylimidazoliumbis(trifluormethylsulfonyl)imid ([C4mim] [Tf2N]), 1-n-Butyl-3-methylimidazoliumbromid ([C4mim] [Br]), 1-Ethyl-3-methylimidazoliumhexafulorophosphat ([C2mim] [PF6]), 1-Ethyl-3-methylimidazoliumbis(trifluormethylsulfonyl)imid ([C2mim] [Tf2N]), 1-Hexyl-3-methylimidazoliumtetrafuloroborat ([C6mim] [BF4]), 1-Hexyl-3-methylimidazoliumhexafulorophosphat ([C6mim] [PF6]), 1-Hexyl-3-methylimidazoliumbis(trifluormethylsulfonyl)imid ([C6mim][Tf2N]), 1-Octyl-3-methylimidazoliumtetrafluorborat ([C8mim] [BF4]), 1-Octyl-3-methylimidazolium-bis(trifluormethylsulfonyl)imid ([C8mim] [Tf2N]), Trihexyl(tetradecyl)phosphoniumpyrazol ([P66614][Pyr]), Trihexyl(tetradecyl)phosphoniumimidazol ([P66614][lm]), Trihexyl(tetradecyl)phosphoniumindol ([P66614][Ind]), Trihexyl(tetradecyl)phosphoniumtrizol ([P66614][Triz]), Trihexyl(tetradecyl)phosphoniumbentrizol ([P66614][Bentriz]), Trihexyl(tetradecyl)phosphoniumtetrazol ([P66614][Tetz]), und Trihexyl(tetradecyl)phosphoniumbromid ([P66614][Br]) ausgewählt ist.

5. Verfahren nach Anspruch 1, wobei die Farbwechsel-Farbstoffe eine Substanz umfassen, die mindestens eine charakteristische Änderung der Farbe, des Chromas, des Lumas und der Wahrnehmung aufweist, die einer Frequenzänderung einer Wellenlänge innerhalb des sichtbaren Bereichs, vom sichtbaren Bereich in den infraroten oder ultravioletten Bereich, vom infraroten oder ultravioletten Bereich in den sichtbaren Bereich oder einer Intensitätsänderung einer Wellenlänge bei Reaktion mit den Molekülen des jeweiligen Gases zuzuschreiben ist.

6. Verfahren nach Anspruch 1, wobei mindestens einer der Farbwechsel-Farbstoffe ausgewählt ist aus einer Gruppe bestehend aus Blei(II)-acetat(Pb(CH3COO)2), Eisen(II)-acetat(Fe(CH3COO)2), Nickel(II)-acetat(Ni(CH3COO)2), Kupfer(II)-acetat(Cu(CH3COO)2), Cadmiumacetat(Cd(CH3COO)2), Kobalt(II)-acetat(Co(CH3COO)2), Mangan(II)-acetat (Cu(CH3COO)2), Bismut(III)-acetat(Co(CH3COO)3), Silber(I)-acetat(Ag(CH3COO)), Silbernitrid (AgNO3), Otolidin, m-Tolidin, Bromphenolblau + TBAH, Methylrot + TBAH, Thymolblau + TBAH, Fluorescein, Broml<resolviolett, Bromphenolrot, LiNO3, 5-10-15-20-Tetraphenylporphyrinatozinl< (II) und 5-10-15-20-Tetral<is(2,4,6-Trimethylphenyl)porphyrinatozinl< (II).

7. Verfahren nach Anspruch 1, wobei die Farbwechsel-Farbstoffe einen Durchmesser von 1 nm ~ 1 µm haben.

8. Verfahren nach Anspruch 1, wobei die Polymer-Nanofaser durch Elektrospinnen einer Polymerlösung gebildet wird, die die ionischen Flüssigkeiten und die Farbwechsel-Farbstoffe enthält.

9. Das Verfahren nach Anspruch 1, wobei:
die Polymer-Nanofaser einen Durchmesser von 100 nm ~ 10 µm hat,
und
die Garnstruktur einen Durchmesser von 10 µm ~ 1000 µm hat.

10. Verfahren nach Anspruch 1, wobei der Träger einen Durchmesser von 1 ~ 5.000 µm und eine Zugfestigkeit von 50 ~ 3.000 MPa aufweist.

11. Verfahren nach Anspruch 1, wobei der Träger mindestens eines der Metalle, ausgewählt aus einer Gruppe bestehend aus Fe, W, Ti, Cu, Ni, Zn und rostfreiem Stahl, eine Naturfaser, ausgewählt aus einer Gruppe bestehend aus Baumwolle, Leinen, Seide und Wolle, und eine Chemiefaser, ausgewählt aus einer Gruppe bestehend aus Nylon, Polyester, Acryl, Polyvinylalkohol, Polyvinylloid, Polyethylen, Polypropylen, Polyurethan, Rayon, einer Acetatglasfaser und einer Metallfaser, umfasst.

12. Verfahren nach Anspruch 1, wobei das Gewichtsverhältnis der ionischen Flüssigkeiten 0,1 Gew.-% bis 100 Gew.-% im Vergleich zum Gewicht des in der Polymer-Nanofaser verwendeten Polymers beträgt.

13. Verfahren nach Anspruch 1, wobei das Gewichtsverhältnis der Farbwechsel-Farbstoffe einen Konzentrationsbereich von 0,1 Gew.-% bis 400 Gew.-%, bezogen auf das Gewicht des in der Polymer-Nanofaser verwendeten Polymers, aufweist.

14. Verfahren nach Anspruch 1, wobei ein Polymer, das die Polymer-Nanofaser konfiguriert, mindestens eines umfasst, das aus einer Gruppe ausgewählt ist, die aus Polyperfurylall<ohol (PPFA), Polymethylmethacrylat (PMMA), Polyacryl-Copolymer, Polyvinylacetat (PVAc), Polyvinylacetat-Copolymer, Polystyrol (PS), Polyvinylpyrrolidon (PVP), Polystyrol-Copolymer, Polyethylenoxid (PEO), Polyethylenoxid-Copolymer, Polycarbonat (PC), Polyvinylchlorid (PVC), Polypropylenoxid-Copolymer, Polycaprolacton, Polyvinylfluorid, Polyvinyllidenfluorid (Poly(vinylidenfluorid) (PVDF)), Polyvinyllidenfluorid-Copolymer, Polyimid, Polyacrylnitril (PAN), 73-49 Polyethylenterephthalat (PET), Polypropylenoxid (PPO), Polyvinylalkohol (PVA), Styrol-Acrylnitril (SAN), Polycarbonat (PC), Polyanilin (PANI), Polypropylen (PP) und Polyethylen (PE) besteht.

## Revendications

1. Procédé de fabrication d'un capteur de gaz, comprenant les étapes suivantes:
(a) la fabrication d'une solution mixte dans laquelle les liquides ioniques et les colorants à changement de couleur sont mélangés avec la solution de polymère dans laquelle un polymère est dissous dans un solvant ;
(b) dissoudre les liquides ioniques et les colorants à changement de couleur dans la solution mélangée par un processus d'agitation à haute température, où la haute température est à une température d'un point de fusion des colorants à changement de couleur ou plus;
(c) la fabrication d'une solution d'électrofilage contenant des colorants recristallisés en fins cristaux par un processus de trempe de la solution mixte dans laquelle les liquides ioniques et les colorants de changement de couleur ont été dissous ;
(d) la fabrication d'une nanofibre polymère unidimensionnelle (1-D) dans laquelle les liquides ioniques et les colorants de changement de couleur ont été ancrés en utilisant un procédé d'électrofilage double et la fabrication de la nanofibre polymère 1-D en une nanofibre ayant une structure de réseau 3-D en forme de fil ; et
(e) enrouler et collecter la nanofibre avec la structure de réseau tridimensionnelle d'une forme de fil en utilisant un enrouleur.

2. Procédé de la revendication 1, dans lequel la structure de réseau 3D est une structure de membrane poreuse 3D dans laquelle les nanofibres de polymère ayant une structure de forme 1-D sont enchevêtrées de manière aléatoire sur le support.

3. Procédé de la revendication 1, dans lequel :
les liquides ioniques ont une solubilité élevée pour le gaz spécifique, et
les liquides ioniques ont une pression de vapeur de 10⁻⁹ ~ 10⁻¹² Pa à température ambiante et restent à la surface de la nanofibre polymère et sont fonctionnalisés sans s'évaporer même après l'électrofilage.

4. Procédé selon la revendication 1, dans lequel au moins un des liquides ioniques est choisi dans le groupe constitué par le tétrafuloroborate de 1-n-butyl-3-méthylimidazolium ([C4mim] [BF4]), l'hexafulorophosphate de 1-n-butyl-3-méthylimidazolium ([C4mim] [PF6]), le bis(trifluorométhylsulfonyl)imide de 1-n-butyl-3-méthylimidazolium ([C4mim] [Tf2N]), bromure de 1-n-butyl-3-méthylimidazolium ([C4mim] [Br]), hexafulorophosphate de 1-éthyl-3-méthylimidazolium ([C2mim] [PF6]), bis(trifluorométhylsulfonyl)imide de 1-éthyl-3-méthylimidazolium ([C2mim] [Tf2N]), tétrafuloroborate de 1-hexyl-3-méthylimidazolium ([C6mim] [BF4]), hexafulorophosphate de 1-hexyl-3-méthylimidazolium ([C6mim] [PF6]), bis(trifluorométhylsulfonyl)imide de 1-hexyl-3-méthylimidazolium ([C6mim] [Tf2N]), tétrafluoroborate de 1-octyl-3-méthylimidazolium ([C8mim] [BF4]), bis(trifluorométhylsulfonyl)imide de 1-octyl-3-méthylimidazolium ([C8mim] [Tf2N]), pyrazole de trihexyl(tétradécyl)phosphonium ([P66614][Pyr]), imidazole de trihexyl(tétradécyl)phosphonium ([P66614][Im]), indole de trihexyl(tétradécyl)phosphonium ([P66614][Ind]), trihexyl(tétradécyl)phosphonium Trizole ([P66614][Triz]), trihexyl(tétradécyl)phosphonium bentrizole ([P66614][Bentriz]), le tétrazole de trihexyl(tétradécyl)phosphonium ([P66614][Tetz]), et le bromure de trihexyl(tétradécyl)phosphonium ([P66614][Br]).

5. Procédé de la revendication 1, dans lequel les colorants à changement de couleur comprennent une substance ayant au moins un changement caractéristique de couleur, de chroma, de luma et de perception attribuable à un changement de fréquence d'une longueur d'onde dans la gamme visible, de la gamme visible à la gamme infrarouge ou ultraviolette, de la gamme infrarouge ou ultraviolette à la gamme visible, ou un changement d'intensité d'une longueur d'onde lors de la réaction avec les molécules du gaz spécifique.

6. Procédé de la revendication 1, dans lequel au moins un des colorants de changement de couleur est choisi dans un groupe constitué par l'acétate de plomb(II)(Pb(CH3COO)2), l'acétate de fer(II)(Fe(CH3COO)2), acétate de nickel(II)(Ni(CH3COO)2), acétate de cuivre(II)(Cu(CH3COO)2), acétate de cadmium(Cd(CH3COO)2), acétate de cobalt(II)(Co(CH3COO)2), acétate de manganèse(II)(Cu(CH3COO)2), acétate de bismuth(III)(Co(CH3COO)3), acétate d'argent(I)(Ag(CH3COO)), nitrure d'argent (AgNO3), otolidine, m-tolidine, bleu de bromophénol + TBAH, rouge de méthyle + TBAH, bleu de thymol + TBAH, fluorescéine, pourpre de bromocrésol, rouge de bromophénol, LiNO3, 5-10-15-20-tétraphénylporphyrinatozinc (II), et 5-10-15-20-tétrakis(2,4,6-triméthylphényl)porphyrinatozinc (II).

7. Le procédé de la revendication 1, dans lequel les colorants de changement de couleur ont un diamètre de 1 nm ~ 1 µm.

8. Procédé de la revendication 1, dans lequel la nanofibre polymère est formée par filage électrique d'une solution polymère comprenant les liquides ioniques et les colorants à changement de couleur.

9. Procédé de la revendication 1, dans lequel :
la nanofibre de polymère a un diamètre de 100 nm ~ 10 µm,
et
la structure du fil a un diamètre de 10 µm ~ 1000 µm.

10. Le procédé de la revendication 1, dans lequel le support a un diamètre de 1 ~ 5 000 µm et a une résistance à la traction de 50 ~ 3 000 MPa.

11. Procédé selon la revendication 1, dans lequel le support comprend au moins un des métaux choisis dans un groupe constitué de Fe, W, Ti, Cu, Ni, Zn et d'acier inoxydable, une fibre naturelle choisie dans un groupe constitué de coton, de lin, de soie et de laine, et une fibre synthétique choisie dans un groupe constitué de nylon, de polyester, d'acrylique, d'alcool polyvinylique, de polyvinylloïde, de polyéthylène, de polypropylène, de polyuréthane, de viscose, d'une fibre de verre acétate et d'une fibre métallique.

12. Procédé de la revendication 1, dans lequel un rapport pondéral des liquides ioniques est de 0,1 % en poids ~ 100 % en poids par rapport au poids du polymère utilisé dans la nanofibre polymère.

13. Procédé de la revendication 1, dans lequel le rapport pondéral des colorants de changement de couleur a une plage de concentration de 0,1 % en poids ~ 400 % en poids par rapport au poids du polymère utilisé dans la nanofibre polymère.

14. Procédé selon la revendication 1, dans lequel un polymère configurant la nanofibre polymère comprend au moins un élément choisi dans le groupe constitué par l'alcool polyperfurylique (PPFA), le polyméthacrylate de méthyle (PMMA), un copolymère polyacrylique, l'acétate de polyvinyle (PVAc), un copolymère d'acétate de polyvinyle, le polystyrène (PS), la polyvinylpyrrolidone (PVP), un copolymère de polystyrène, l'oxyde de polyéthylène (PEO), un copolymère d'oxyde de polyéthylène, le polycarbonate (PC), chlorure de polyvinyle (PVC), copolymère d'oxyde de polypropylène, polycaprolactone, polyfluorure de vinyle, polyfluorure de vinylidène (PVDF), copolymère de polyfluorure de vinylidène, polyimide, polyacrylonitrile (PAN), 73-49 polyéthylène téréphtalate (PET), polypropylène oxyde (PPO), polyvinylalcool (PVA), styrène-acrylonitrile (SAN), polycarbonate (PC), polyaniline (PANI), polypropylène (PP) et polyéthylène (PE).
